# EUROPEAN PATENT SPECIFICATION

(11) **EP 0 497 425 B1**
(45) Date of publication and mention of the grant of the patent: **27.08.1997**
(21) Application number: 92200243.1
(22) Date of filing: 28.01.1992
(51) Int. Cl.: C10J 3/00, C07C 1/04, C10J 3/46, F01K 23/06

(54) **Process for the combined production of organic compounds and power**
Verfahren zur kombinierten Herstellung von organischen Zusammenstellungen und Energie
Procédé pour la production combinée de composés organiques et d'énergie

(30) Priority: 30.01.1991 GB 9101959
(43) Date of publication of application: 05.08.1992
(73) Proprietor: SHELL INTERNATIONALE RESEARCH MAATSCHAPPIJ B.V., 2596 HR Den Haag (NL)
(72) Inventor: Naber, Jaap Eric, NL-1031 CM Amsterdam (NL)

(56) References cited:
- DE-A- 2 460 919
- DE-A- 2 735 829
- DE-A- 3 210 411
- FR-A- 2 512 000
- GB-A- 1 590 706
- NL-A- 7 413 678
- US-A- 3 838 993
- DE-A- 2 460 919
- DE-A- 2 735 829
- DE-A- 3 210 411
- FR-A- 2 512 000
- GB-A- 1 590 706
- NL-A- 7 413 678
- NL-A- 7 413 678
- US-A- 3 838 993

## Description

The present invention relates to a process for the combined production of organic compounds, such as hydrocarbons and methanol, and of power from heavy hydrocarbons comprising pitch.

In conventional refinery operations, desirable products, for example fuels, lubricating oils and chemicals, are prepared from a crude oil feedstock by such processes as fractionation (distillation), thermal cracking, hydrocracking and hydroconversion. From an economic standpoint, it is important to convert as much as possible of the crude oil into valuable end products. Nevertheless, the refinery operations yield less valuable heavy hydrocarbon by-products, for example pitch and both atmospheric and vacuum residues. Accordingly, it would be most advantageous to be able to convert these less valuable, heavy hydrocarbon by-products into more desirable, higher value products, such as those mentioned hereinbefore.

The term "pitch" as used herein is a reference to the residue remaining after the thermal cracking, hydrocracking or hydroconversion of atmospheric or vacuum residues.

US patent specification number 4,433,065 (US-A-4,433,065) discloses a process in which a carbon-containing starting material, most especially coal, is converted in a gasifying reactor into a gaseous mixture comprising carbon monoxide and hydrogen. The resulting gaseous mixture is then passed to a hydrocarbon synthesis reactor in which the carbon monoxide and hydrogen are converted into hydrocarbons. The gaseous effluent of the hydrocarbon synthesis reactor, comprising unconverted carbon monoxide and hydrogen may be recycled. Alternatively, US-A-4,433,056 discloses that the effluent gas may be passed to a back-pressure turbine, thereby generating electricity, and thereafter combusted. The hot combustion gases are used to generate power by means of a gas turbine.

A similar process to that of US-A-4,433,605 is described in European patent number 0 128 404 (EP-B-0 128 404), in which coal is first gasified to yield a mixture of carbon monoxide and hydrogen, which mixture is used as a starting material in the synthesis of methanol, prior to being passed to the combustion chamber of a gas turbine.

Further, European patent number 0 103 914 (EP-B-0 103 914) discloses a process for the combined generation of power and the preparation of hydrocarbons, in which a mixture of carbon monoxide and hydrogen is partly converted into the desired hydrocarbons, with the remainder being combusted for use in the generation of power by means of a gas turbine. The mixture of carbon monoxide and hydrogen is described in EP-B-0 103 914 to be very suitably obtained by the gasification of carbonaceous materials, such as brown coal, anthracite, coke, crude mineral oil and fractions thereof, as well as oils produced from tar sand and bituminous shale. The most preferred starting material for the preparations of the carbon monoxide and hydrogen mixture is stated in EP-B-0 103 914 to be coal.

Accordingly, it can be seen that much effort has been devoted to the generation of valuable hydrocarbon products from lower value hydrocarbonaceous starting materials, most specifically coal. There remains, however, a need for an efficient process for the conversion of pitch and the atmospheric and vacuum residues mentioned hereinbefore into valuable products.

One solution to this problem has been proposed in the specification of European patent number 0 142 889 (EP-B-0 142 889). EP-B-0 142 889 discloses a process for the preparation of hydrocarbons and fuel gas from a heavy carbonaceous feedstock, for example long or short (atmospheric or vacuum) residues, pitch, asphalt, bitumen, tar, shale oil, coal liquefication products and slurried solid fuels, such as coal, comprising cracking the feedstock in a coking zone to yield hydrocarbons and coke, gasifying the coke to yield carbon monoxide and hydrogen, converting the carbon monoxide and hydrogen so-produced into hydrocarbons and a fuel gas comprising the light hydrocarbon products and unconverted carbon monoxide and hydrogen.

Surprisingly, a simple process scheme has been found which allows the conversion of pitch and atmospheric and vacuum residues, hereinafter referred to as "heavy hydrocarbons", into more valuable hydrocarbons, whilst at the same time allowing the generation of power, without the need for a coking step.

Accordingly, the present invention provides a process for the combined production of organic compounds and power from heavy hydrocarbons, which process comprises the steps of:
i) gasifying the heavy hydrocarbons to a gaseous mixture comprising hydrogen and carbon monoxide;
ii) partially, catalytically converting the gaseous mixture into organic compounds using a catalyst; and
iii) generating power from the unconverted gaseous mixture using a gas turbine/steam turbine cycle.

The process may conveniently and advantageously be operated in a single pass regime devoid of any recycle streams, thus allowing the processing to be comparatively simple and at relatively low cost.

The valuable products produced are the organic compounds obtained by the catalytic conversion of the gaseous mixture of carbon monoxide and hydrogen. When the catalytic conversion produces predominantly waxes, these products may be cracked to yield products such as gas oil and gasoline. Optionally, when the process comprises a hydrogen extraction unit, the process according to the invention further provides hydrogen gas as a valuable product.

The heavy hydrocarbons used in the gasification step most preferably comprise pitch. Preferably the pitch has been obtained from thermally cracking residues to a 520+ °C conversion of over 25 %wt, preferably over 50 %wt. The pitch should have a suitable viscosity. Generally the viscosity is preferably higher than 2000 mm²/s (cSt) (150 °C), more preferably higher than 40,000 mm²/s (cSt) (150 °C). The amount of coke present in the pitch should be low, generally less than 10% by weight, preferably less than 5% by weight. The pitch used in the gasification stage of the process preferably has a hydrogen to carbon ratio such that the gaseous mixture produced by gasification is suitable for the production of organic compounds, in particular paraffinic and aromatic hydrocarbons and methanol.

The pitch may be formed by thermal cracking of vacuum residue or atmospheric residue. These residues are obtained by the vacuum or atmospheric distillation of crude oil. Accordingly, a less valuable by-product from the refining of crude oil is used as a starting material for the preparation of a valuable end product. Atmospheric residue has an initial boiling point higher than about 350 °C and vacuum residue has an initial boiling point in the range of about 500 to 550 °C. These residues may be converted into pitch by thermal cracking, such as furnace cracking and soaker cracking. Furnace cracking comprises cracking of residue at about 500 °C for approximately 5 minutes followed by cooling and removal by distillation of gas, gasoline and gas oil. Soaker cracking comprises cracking at less than 500 °C for about 0.5 to 3 minutes followed by a hold up in a soaker vessel for 10 to 30 minutes during which hold up time the temperature decreases by about 30 °C. Finally, gas, gasoline and gas oil are removed by distillation.

The gasification of the heavy hydrocarbons is carried out in a gasification zone, preferably by partial oxidation using an oxygen-containing gas. The oxygen-containing gas may be air, oxygen-enriched air or oxygen. Preferably, the oxygen-containing gas is oxygen-enriched air. The oxygen-containing gas may be mixed with steam.

Steam may be introduced into the gasification zone together with or apart from the oxygen-containing gas. The oxygen-containing gas and if appropriate steam, together with the heavy hydrocarbons are fed to the burner of the gasification reactor, where they are mixed and the hydrocarbons gasified. The gasification is typically carried out at a temperature of from about 800 to 1500 °C, preferably about 1200 to 1500 °C, and a pressure of from 1 to 15 bar when using air, ranging up to 60 bar when using oxygen. Any coke present is almost substantially gasified.

Typically the gaseous mixture formed during the gasification stage comprises a hydrogen to carbon monoxide ratio in the range of about 1:1 to 1:2.5, preferably about 1:2.

The gaseous mixture formed during the gasification of the heavy hydrocarbons comprises hydrogen, carbon monoxide, carbon dioxide, steam, nitrogen, hydrogen sulphide. Prior to contacting this gaseous mixture with a catalyst for the conversion of this mixture into organic compounds, it is preferred to remove at least most of the hydrogen sulphide, to avoid poisoning the synthesis catalyst. The gaseous mixture may be desulphurized using a conventional desulphurization process. Particularly suitable are processes in which hydrogen sulphide and optionally a part of the carbon dioxide present in the gaseous mixture are absorbed by an absorbing liquid. Very suitable processes include the ADIP and Sulfinol processes, described in the British patent specifications numbers 1,131,989, 972,140, 965,358, 957,260 and 879,090.

The purified gaseous mixture, comprising predominantly hydrogen and carbon monoxide, is contacted with a catalyst in the catalytic conversion stage, by which these compounds are converted into organic compounds. These organic compounds may comprise hydrocarbons, particularly paraffinic and aromatic hydrocarbons, methanol.

The catalysts used for the catalytic conversion of the mixture comprising hydrogen and carbon monoxide into hydrocarbons are known in the art and are referred to as Fischer-Tropsch catalysts. Catalysts for use in the Fischer-Tropsch synthesis process frequently comprise, as the catalytically active component, a metal from Group VIII of the Periodic Table of Elements. Particular catalytically active metals include iron, cobalt and nickel. Cobalt is a preferred catalytically active metal.

The catalytically active metal is preferably supported on a porous carrier. The porous carrier may be selected from any of the suitable refractory metal oxides or silicates or combinations thereof known in the art. Particular examples of preferred porous carriers include silica, alumina, titania, zirconia, ceria, gallia and mixtures thereof. Silica is a particularly preferred carrier material for the catalyst.

The amount of catalytically active metal on the carrier is preferably in the range of from 3 to 300 pbw per 100 pbw of carrier material, more preferably from 10 to 80 pbw, especially from 20 to 60 pbw.

If desired, the catalyst may also comprise one or more metals or metal oxides as promoters. Suitable metal oxide promoters may be selected from Groups IIA, IIIB, IVB, VB and VIB of the Periodic Table of Elements, or the actinides and lanthanides. In particular, oxides of magnesium, calcium, strontium, barium, scandium, yttrium. lanthanum, cerium, titanium, zirconium, hafnium, thorium, uranium, vanadium and chromium are most suitable promoters. A particularly preferred metal oxide promoter for the catalyst used to prepare the waxes for use in the present invention is zirconium oxide. Suitable metal promoters may be selected from Groups VIIB or VIII of the Periodic Table. Rhenium and Group VIII noble metals are particularly suitable, with platinum and palladium being especially preferred. The amount of promoter present in the catalyst is preferably in the range of from 0.01 to 100 pbw per 100 pbw of carrier.

The catalytically active metal and the promoter, if present, may be deposited on the carrier material by any suitable treatment, such as impregnation, kneading and extrusion. After deposition of the metal and, if appropriate, the promoter on the carrier material, the loaded carrier is typically subjected to calcination at a temperature of generally from 350 to 750 °C, preferably a temperature in the range of from 450 to 550 °C. The effect of the calcination treatment is to remove crystal water, to decompose volatile decomposition products and to convert organic and inorganic compounds to their respective oxides. After calcination, the resulting catalyst may be activated by contacting the catalyst with hydrogen or a hydrogen-containing gas, typically at temperatures of about 200 to 350 °C.

A particularly suitable Fischer-Tropsch catalyst is a cobalt/zirconium/silica catalyst. Examples of suitable catalysts which may be used in the preparation of the waxes are disclosed in European Patent Applications publication numbers EP 0 104 672, EP 0 110 449, EP 0 127 220, EP 0 167 215, EP 0 180 269 and EP 0 221 598.

The catalytic conversion process may be performed under conventional synthesis conditions known in the art. Typically, the catalytic conversion may be effected at a temperature in the range of from 100 to 600 °C, preferably from 150 to 350 °C, more preferably from 180 to 270 °C. Typical total pressures for the catalytic conversion process are in the range of from 1 to 200 bar absolute, more preferably from 10 to 70 bar absolute.

Preferably, a Fischer-Tropsch catalyst is used, which yields substantial quantities of paraffins, more preferably substantially unbranched paraffins, of which a considerable portion boils above the boiling point range of the so-called middle distillates. A most suitable catalyst for this purpose is a cobalt-containing Fischer-Tropsch catalyst. The term "middle distillates", as used herein, is a reference to hydrocarbon mixtures of which the boiling point range corresponds substantially to that of kerosine and gas oil fractions obtained in a conventional atmospheric distillation of crude mineral oil. The boiling point range of middle distillates generally lies within the range of about 150 to about 360 °C.

The higher boiling range paraffinic hydrocarbons may be subjected to a catalytic hydrocracking, which is known per se in the art, to yield the desired middle distillates. The catalytic hydrocracking is carried out by contacting the paraffinic hydrocarbons at elevated temperature and pressure and in the presence of hydrogen with a catalyst containing one or more metals having hydrogenation activity, and supported on a carrier. Suitable hydrocracking catalysts include catalysts comprising metals selected from Groups VIB and VIII of the Periodic Table of Elements. Preferably, the hydrocracking catalysts contain one or more noble metals from group VIII. Preferred noble metals are platinum, palladium, rhodium, ruthenium, iridium and osmium. Most preferred catalysts for use in the hydrocracking stage are those comprising platinum.

The amount of catalytically active metal present in the hydrocracking catalyst may vary within wide limits and is typically in the range of from about 0.05 to about 5 parts by weight per 100 parts by weight of the carrier material.

Suitable conditions for the catalytic hydrocracking are known in the art. Typically, the hydrocracking is effected at a temperature in the range of from about 175 to 400 °C. Typical hydrogen partial pressures applied in the hydrocracking process are in the range of from 10 to 250 bar.

As an alternative to preparing substantially paraffinic hydrocarbons using the Fischer-Tropsch catalysts described hereabove, the gaseous mixture of carbon monoxide and hydrogen may be converted, using a bifunctional catalyst, in a first step into acyclic hydrocarbons and/or acyclic oxygen containing hydrocarbons. The product formed is at least partially converted in a second step into aromatic hydrocarbons suitable as high octane gasoline and chemical intermediates.

Suitable bifunctional catalysts are known in the art. Examples of suitable bifunctional catalysts are catalysts comprising a first catalyst component having activity for the conversion of synthesis gas into acyclic hydrocarbons and/or acyclic oxygen-containing hydrocarbons, such as methanol and dimethyl ether, in combination with a second catalyst component having activity for the conversion of the acyclic compounds mentioned into aromatic hydrocarbons.

The first catalyst component of such a bifunctional catalyst typically comprises at least one metal from the iron group of metals or ruthenium, optionally in association with one or more promoters to increase the activity and/or selectivity. The active metal and any promoters may be supported on a carrier material, such as kieselguhr. A specific first catalyst component for use in the catalytic conversion process comprises from 30 to 75 parts by weight iron and from 5 to 40 parts by weight magnesium per 100 parts by weight alumina. Further preferred catalysts for use as the first catalyst component are ZnO/Cr₂O₃ compositions, in particular such compositions in which the atomic percentage of zinc, based on the sum of zinc and chromium, is at least 60%, and preferably from 60 to 80%.

Processes for the preparation of the aforementioned catalysts for use as the first catalyst component are similar to those for Fischer-Tropsch catalysts mentioned above. Details of the catalysts and the processes for their preparation may be found in the art, for example US patent number 4,338,089.

Suitable examples of the second catalyst component for the production of aromatic hydrocarbons are crystalline silicates, for instance crystalline aluminium silicates (zeolites), crystalline iron silicates and crystalline gallium silicates. Details of the aforementioned second catalysts components and the processes for their preparation are again well known in the art.

The catalytic conversion process using the bifunctional catalysts described hereinbefore may be carried out at temperatures in the range of from 200 to 500 °C, preferably from 250 to 450 °C. Typical process operating pressures are in the range of from 1 to 150 bar, preferably from 5 to 100 bar. The process may be operated at a gas hourly space velocity (GHSV) in the range of from 50 to 5000 N1/1/h, preferably from 300 to 3000 N1/1/h.

As a further alternative for the catalytic conversion stage of the process of the present invention, the gaseous mixture of hydrogen and carbon monoxide may serve as a starting material for the preparation of methanol, using any suitable catalytic methanol synthesis process.

The process for the preparation of methanol may employ a catalyst system obtainable by the combination of at least:
i) a salt containing a cation of a metal of Group VIII of the Periodic Table of Elements; and
ii) an alcoholate from an alkali metal or alkaline earth metal.

When using a process employing the above-described catalyst system, the gaseous mixture should not contain substantial amounts of carbon dioxide and/or hydrogen sulphide. Preferably the gaseous mixture does not contain more than 2% by volume carbon dioxide and no more than 0.5% by volume hydrogen sulphide.

Optionally the catalyst system may further comprise:
iii) a hydrate of an alkaline metal or an alkaline earth metal; and
iv) an alcohol or an in situ alcohol-providing agent.

The latter may be any compound which forms in situ an alcohol under the conditions prevailing during the activation of the catalyst and/or the synthesis of the methanol. The in situ alcohol-providing agent is preferably selected from alkyl formates, alkyl oxalates or alkyl carbonates or mixtures thereof, with formates being particularly preferred. According to an alternative embodiment, component i) has the form of a substantially dry salt and the in situ formation of alcohol may be achieved by addition of a relatively small predetermined amount of water.

The elements of Group VIII of the Periodic Table of Elements which may be used as component i) are preferably nickel, cobalt, rhodium, palladium, with nickel being especially preferred.

The alcohol to be used is preferably an alkanol. Preferred are alkanols having 4 to 20 carbon atoms per molecule, such as tertiary butylalcohol, tertiary pentylalcohol, hexanol and heptanol. Dihydric alcohols may be used as such or in combination with alkanols.

The alcoholate to be used is preferably sodium alcoholate or potassium alcoholate. Preferred alcoholates are alkoxides, particularly those having 1 to 20 carbon atoms per molecule, such as sodium methoxide, potassium methoxide, sodium ethoxide, sodium butoxide, potassium butoxide, potassium isobutoxide, sodium isobutoxide, sodium tertiary pentoxide, potassium tertiary pentoxide and potassium 2-methyl dodeca-2-oxide.

The anion of the salt of component i) may be derived from a large variety of acids depending on the specific embodiments of the catalyst system. In a preferred embodiment, nickel formate, nickel acetate, nickel oxalate or nickel tosylate are used.

Most preferably, however, when a process for the preparation of methanol is the catalytic conversion process of the process of the present invention, a catalyst comprising oxides of zinc and chromium, oxides of zinc and aluminium, oxides of copper, chromium and aluminium, or oxides of copper, aluminium and zinc is employed. The oxides may be supported on any suitable carrier material, for example any of those mentioned above in connection with the catalysts of the alternative catalytic conversion processes. The catalyst most preferably comprises oxides of copper and zinc on an alumina support.

In this most preferred methanol synthesis process, the mixture of carbon monoxide and hydrogen may be contacted with the catalyst at a temperature in the range of from 150 to 350 °C, preferably from 200 to 300 °C. Typical operating pressures for this process are in the range of from 1 to 100 bar, preferably from 10 to 80 bar. Details of this preferred process for the preparation of methanol are known in the art, for example from US patent numbers 4,481,305 and 4,520,216.

It will be appreciated that the catalytic conversion stage of the process of the present invention may comprise one or a combination of the alternative catalytic conversion processes hereinbefore described.

After the partial catalytic conversion of the mixture of carbon monoxide and hydrogen into organic compounds, the remaining gaseous mixture may comprise relatively large amounts of hydrogen. Optionally, hydrogen may be extracted from the gaseous mixture using any suitable technique, such as membrane diffusion. Prior to the hydrogen extraction the amount of hydrogen in the gaseous mixture may be increased at the expense of carbon monoxide using the water gas shift reaction. This water gas shift reaction may suitably be carried out by contacting the gaseous mixture, together with from 1 to 3 mol of steam per mol carbon monoxide with a catalyst containing metals having water gas shift activity, such as iron, or copper and zinc. The water gas shift reaction may be effected at a temperature in the range of from 200 to 500 °C Suitable pressures for carrying out the water gas shift are typically in the range of from 10 to 50 bar. The water gas shift reaction may very suitably be carried out in two steps, a first step at a high temperature, for example from 350 to 450 °C and a second step at a low temperature, for example from 200 to 300 °C.

The gaseous mixture remaining after the above-described processes still contains carbon monoxide, hydrogen and light hydrocarbons, the heating value of which as fuel gas is between 3 and 10 MJ/N m³. Accordingly, the remaining gas mixture is an excellent material for use in the generation of power.

An attractive method of generating power and that forming the power generation stage of the process of the present invention is a combined gas turbine/steam turbine cycle. In short, the principle of operation of the gas turbine/steam turbine system is that the remaining gaseous mixture is first combusted, the combustion gases are then expanded in the gas turbine, thereby generating power, and the hot exhaust gas from the gas turbine is thereafter used in the production of high pressure steam. The steam thus generated is expanded in a steam turbine and produces an additional quantity of power. Turbines of this kind are known in the art.

In a preferred arrangement, part of the steam supply of the steam turbine is provided by steam generated in the gasification stage of the process of the present invention. This steam may be preheated by indirect heat exchange with the hot exhaust gases leaving the gas turbine.

## Claims

1. Process for the combined production of organic compounds and power from heavy hydrocarbons, comprising the steps of:
i) gasifying the heavy hydrocarbons to a gaseous mixture comprising hydrogen and carbon monoxide;
ii) partially, catalytically converting the gaseous mixture into organic compounds using a catalyst; and
iii) generating power from the unconverted gaseous mixture using a gas turbine/steam turbine cycle, characterised in that the heavy hydrocarbons comprise pitch.

2. Process as claimed in claim 1, characterised in that the process is a single pass process devoid of recycle streams.

3. Process as claimed in claim 1, characterised in that the pitch has been obtained from thermally cracking residues to a 520+ °C conversion of over 25 %wt, preferably of over 50 %wt.

4. Process as claimed in any preceding claim, characterised in that the gaseous mixture is partially, catalytically converted into hydrocarbons, preferably substantially paraffinic hydrocarbons or substantially aromatic hydrocarbons, or methanol.

5. Process as claimed in claim 4, characterised in that the gaseous mixture is partially catalytically converted into substantially paraffinic hydrocarbons by contact with a catalyst comprising a metal from Group VIII of the Periodic Table, preferably iron, cobalt or nickel, more preferably cobalt.

6. Process as claimed in either of claims 4 or 5, characterised in that the gaseous mixture is partially catalytically converted into substantially paraffinic hydrocarbons, at least part of which are subjected to hydrocracking to yield middle distillates.

7. Process as claimed in any preceding claim, characterised in that, prior to the power generation, hydrogen is extracted from the gaseous mixture.

8. Process as claimed in claim 7, characterised in that the gaseous mixture is contacted with a catalyst having activity in the water gas shift reaction prior to the extraction of hydrogen.

9. Process as claimed in any preceding claim, characterised in that the steam supply to the steam turbine of the gas turbine/steam turbine cycle is supplemented by steam generated in the gasification stage.

## Patentansprüche

1. Verfahren zur kombinierten Herstellung von organischen Verbindungen und Strom aus schweren Kohlenwasserstoffen, bei dem man:
i) die schweren Kohlenwasserstoffe zu einer Wasserstoff und Kohlenmonoxid enthaltenden gasförmigen Mischung vergast;
ii) die gasförmige Mischung unter Verwendung eines Katalysators teilweise katalytisch in organische Verbindungen umwandelt und
iii) aus der nicht umgesetzten gasförmigen Mischung unter Verwendung eines Gasturbinen-Dampfturbinen-Zyklus Strom erzeugt,
dadurch gekennzeichnet, daß die schweren Kohlenwasserstoffe Pech enthalten.

2. Verfahren nach Anspruch 1, dadurch gekennzeichnet, daß es sich dabei um ein Durchlaufverfahren ohne Rückführströme handelt.

3. Verfahren nach Anspruch 1, dadurch gekennzeichnet, daß das Pech aus dem thermischen Cracken von Rückständen bis zu einem 520+ °C-Umsatz von über 25 Gew.-%, bevorzugt über 50 Gew.-%, erhalten wurde.

4. Verfahren nach einem der vorhergehenden Ansprüche, dadurch gekennzeichnet, daß man die gasförmige Mischung teilweise katalytisch in Kohlenwasserstoffe, bevorzugt im wesentlichen paraffinische Kohlenwasserstoffe oder im wesentlichen aromatische Kohlenwasserstoffe, oder Methanol umwandelt.

5. Verfahren nach Anspruch 4, dadurch gekennzeichnet, daß man die gasförmige Mischung teilweise katalytisch zu im wesentlichen paraffinischen Kohlenwasserstoffen umwandelt, indem man sie mit einem ein Metall der Gruppe VIII des Periodensystems, bevorzugt Eisen, Cobalt oder Nickel, besonders bevorzugt Cobalt, enthaltenden Katalysator in Berührung bringt.

6. Verfahren nach Anspruch 4 oder 5, dadurch gekennzeichnet, daß man die gasförmige Mischung teilweise katalytisch in im wesentlichen paraffinische Kohlenwasserstoffe umwandelt, die man mindestens teilweise einem Hydrocrackverfahren zur Herstellung von Mitteldestillaten unterwirft.

7. Verfahren nach einem der vorhergehenden Ansprüche, dadurch gekennzeichnet, daß man vor der Stromerzeugung aus der gasförmigen Mischung Wasserstoff extrahiert.

8. Verfahren nach Anspruch 7, dadurch gekennzeichnet, daß man die gasförmige Mischung vor der Wasserstoffextraktion mit einem Katalysator in Berührung bringt, der bei der Wassergas-Konvertierung aktiv ist.

9. Verfahren nach einem der vorhergehenden Ansprüche, dadurch gekennzeichnet, daß man die Dampfzufuhr zur Dampfturbine des Gasturbinen-Dampfturbinen-Zyklus durch in der Vergasungsstufe erzeugten Dampf ergänzt.

## Revendications

1. Procédé de production combinée de composés organiques et d'énergie à partir d'hydrocarbures lourds, comprenant les étapes consistant à :
i) gazéifier les hydrocarbures lourds en un mélange gazeux comprenant de l'hydrogène et du monoxyde de carbone,
ii) convertir catalytiquement partiellement le mélange gazeux en composés organiques en utilisant un catalyseur, et
iii) générer de l'énergie à partir du mélange gazeux non converti en utilisant un cycle turbine à gaz/turbine à vapeur,
caractérisé en ce que les hydrocarbures lourds comprennent du brai.

2. Procédé suivant la revendication 1, caractérisé en ce que le procédé est un procédé à passe unique dépourvu de courant de recyclage.

3. Procédé suivant la revendication 1, caractérisé en ce que le brai a été obtenu à partir de résidus de craquage thermique à une conversion de 520+°C supérieure à 25% en poids de préférence, supérieure à 50% en poids.

4. Procédé suivant l'une quelconque des revendications précédentes, caractérisé en ce que le mélange gazeux est catalytiquement partiellement converti en hydrocarbures, de préférence, des hydrocarbures sensiblement paraffiniques, ou des hydrocarbures sensiblement aromatiques, ou du méthanol.

5. Procédé suivant la revendication 4, caractérisé en ce que le mélange gazeux est partiellement catalytiquement converti en hydrocarbures sensiblement paraffiniques par mise en contact avec un catalyseur comprenant un métal appartenant au groupe VIII du tableau périodique des éléments, de préférence, le fer, le cobalt ou le nickel, plus avantageusement, le cobalt.

6. Procédé suivant l'une quelconque des revendications 4 et 5, caractérisé en ce que le mélange gazeux est partiellement catalytiquement converti en hydrocarbures sensiblement paraffiniques, dont au moins une partie est soumise à un hydrocraquage pour donner des distillats moyens.

7. Procédé suivant l'une quelconque des revendications précédentes, caractérisé en ce que, préalablement à la génération d'énergie, on extrait de l'hydrogène du mélange gazeux.

8. Procédé suivant la revendication 7, caractérisé en ce que l'on met le mélange gazeux en contact avec un catalyseur possédant une activité dans la réaction de déplacement eau-gaz avant de procéder à l'extraction de l'hydrogène.

9. Procédé suivant l'une quelconque des revendications précédentes, caractérisé en ce que la fourniture de vapeur d'eau à la turbine à vapeur du cycle turbine à gaz/turbine à vapeur provient de la vapeur d'eau engendrée au cours de l'étape de gazéification.
